(19)

Europäisches
Patentamt
European
Patent Office
Office européen
des brevets

(11) EP 2 239 568 A1

(12) EUROPEAN PATENT APPLICATION

(43) Date of publication:
13.10.2010 Bulletin 2010/41

(51) Int Cl.:
G01N 33/493 (2006.01)   G01N 33/68 (2006.01)

(21) Application number: 10158139.5

(22) Date of filing: 29.03.2010

(84) Designated Contracting States:
AT BE BG CH CY CZ DE DK EE ES FI FR GB GR
HR HU IE IS IT LI LT LU LV MC MK MT NL NO PL
PT RO SE SI SK SM TR
Designated Extension States:
AL BA ME RS

(30) Priority: 31.03.2009 JP 2009084657

(71) Applicant: SYSMEX CORPORATION
Kobe-shi,
Hyogo 651-0073 (JP)

(72) Inventors:
• Tanaka, Yousuke
  Hyogo 651-0073 (JP)
• Kishi, Kouji
  Hyogo 651-0073 (JP)
• Inoue, Junya
  Hyogo 651-0073 (JP)
• Tsutsumida, Keisuke
  Hyogo 651-0073 (JP)

(74) Representative: HOFFMANN EITLE
Patent- und Rechtsanwälte
Arabellastrasse 4
81925 München (DE)

(54) **Diagnostic support apparatus for renal disease and computer program product**

(57) The present invention is to present a diagnostic support apparatus for renal disease, comprising: a red blood cell information obtainer for obtaining at least one of red blood cell numerical information representing the number of red blood cells in a urine sample, and red blood cell morphological information representing morphology of the red blood cells in the urine sample; a quantitative value information obtainer for obtaining at least one of quantitative value information representing amount of protein component in the urine sample, and quantitative value information representing amount of lipid component in the urine sample; and a diagnostic support information output unit for outputting diagnostic support information related to renal disease based on information obtained by the red blood cell information obtainer and information obtained by the quantitative value information obtainer.

Fig. 1

EP 2 239 568 A1

**Description**

FIELD OF THE INVENTION

The present invention relates to a diagnostic support apparatus and computer program product for outputting diagnostic support information based on the results of measurements of a urine sample.

BACKGROUND

The number of patients suffering from renal dysfunction that leads to dialysis has been increasing in recent years, and there is demand to reduce the number of patients shifting to dialysis by detecting renal disease at an early stage.

The typical flow of discovery of suspected renal disease from some urine analysis results to the confirmation of renal disease may transpire, for example, as described below. At an initial stage, a general practitioner physician who is not a renal disease specialist may perform qualitative measurements of urine components using litmus paper, and follow through to the next stage when some type of disease is suspected from the urine examination results. In this next stage, urine qualitative exams and urine sediment exams are performed repeatedly for formed components (red blood cells and the like) in the urine by a general practitioner physician at another hospital, and the flow moves to the next stage when these results indicate suspected disease of some kind. In this further stage, more precise urine qualitative exams and urine sediment exams are performed by a general practitioner physician at another hospital, and the flow then shifts to diagnosis by a specialist in renal disease when these further exams also indicate suspected renal disease. The nephrologist performs a definitive diagnosis based on the results of the urine sediment exams and results of the urine quantitative exams which are chemical quantitative exams of components (protein and the like) in the urine.

Various types of examination are known in the art of urine sediment exams and urine quantitative exams.

For example, United States Patent No. 5,325,168 discloses a cell analyzer for classifying and counting cells such as white blood cells, red blood cells, epithelial cells, casts, bacteria and the like found in urine using a flow cytometric method.

As a further example, Japanese Patent Publication No. 2001/228158 discloses an automated analyzer for performing quantitative measurements of the protein components and lipid components in urine.

In order to avoid having the disease progress to requiring dialysis treatment, suspected renal disease must be detected at an earlier stage. However, in the flow of diagnosis of renal disease described above, the urine sediment exams and urine qualitative exams are performed many times before the special physician delivers a confirming diagnosis because it is difficult to detect suspected renal disease in the early stage in which the urine qualitative exams and urine sediment exams are performed without the aid of a specialist physician. Therefore, a great deal of time elapses before the specialist physician delivers the confirming diagnosis. A diagnosis by a medical specialist therefore may be difficult in the early stages, and when the confirming diagnosis is made by the specialist the renal disease may already have progressed to near the stage of requiring dialysis treatment. Thus, it heretofore has been difficult to detect suspected renal disease at an early stage because it is difficult to detect suspected renal disease in the examination stages performed by non-specialist physicians.

SUMMARY

A first aspect of the present invention is a diagnostic support apparatus for renal disease, comprising: a red blood cell information obtainer for obtaining at least one of red blood cell numerical information representing the number of red blood cells in a urine sample, and red blood cell morphological information representing morphology of the red blood cells in the urine sample; a quantitative value information obtainer for obtaining at least one of quantitative value information representing amount of protein component in the urine sample, and quantitative value information representing amount of lipid component in the urine sample; and a diagnostic support information output unit for outputting diagnostic support information related to renal disease based on information obtained by the red blood cell information obtainer and information obtained by the quantitative value information obtainer.

According to the structures described above, suspected renal disease can be detected using the provided renal disease diagnostic support information even without a specialist physician because renal disease diagnostic support information is output based on the results (information obtained by the red blood cell information obtainer and quantitative value information obtained by the quantitative value information obtainer) of both the urine sediment exams and urine quantitative exams conventionally performed in the confirming diagnosis by a medical specialist. Therefore, suspected renal disease can be detected by a non-specialist physician when using the renal disease diagnostic support apparatus of the present invention during the diagnostic process for renal disease performed through a plurality of stages, even in the stages prior to the confirming diagnosis by a specialist physician. Suspected renal disease can thus be detected at an early stage.

Preferably, the protein component comprises at least one of total protein and albumin; and the lipid component comprises cholesterol.

More preferably, the renal disease diagnostic support information comprises renal disease diagnostic support information related to suspected renal disease;

and the diagnostic support information output unit outputs the renal disease diagnostic support information related to suspected renal disease, based on the information obtained by the red blood cell information obtainer and the information obtained by the quantitative value information obtainer.

According to this structure, whether renal disease is suspected can be determined based on at least one of albumin quantitative value and total protein quantitative value.

Preferably, the renal disease diagnostic support information related to suspected renal disease comprises renal disease diagnostic support information indicating that renal disease is suspected; the quantitative value information obtainer obtains albumin quantitative value information representing amount of albumin in the urine sample, and total protein quantitative value information representing amount of total protein in the urine sample; and the diagnostic support information output unit outputs the renal disease diagnostic support information indicating that renal disease is suspected, based on the albumin quantitative value information and the total protein quantitative value information.

Preferably, the diagnostic support information output unit is configured to perform operations comprising: obtaining a first numerical value representing the amount of albumin in the urine sample based on the albumin quantitative value information; obtaining a second numerical value representing the amount of total protein in the urine sample based on the total protein quantitative value information; and outputting the renal disease diagnostic support information indicating that renal disease is suspected, when at least either the first numerical value exceeds a first threshold value or the second numerical value exceeds a second threshold value, or when the first numerical value is under the first threshold value, the second numerical value is under the second threshold value and the information obtained by the red blood cell information obtainer meets a predetermined condition.

According to this structure, renal disease diagnostic support information representing least suspected renal disease can be uniformly output.

Preferably, the diagnostic support information output unit outputs the renal disease diagnostic support information indicating that renal disease is suspected, when the first numerical value is under the first threshold value, the second numerical value is under the second threshold value, the number of the red blood cells represented by the red blood cell numerical information exceeds a third threshold value and the morphology of the red blood cells represented by the red blood cell morphologic information does not indicate an approximately uniform morphology.

Preferably, the renal disease diagnostic support information indicating that renal disease is suspected comprises at least one of information indicating a degree of suspicion of renal disease and information related to a progression of renal disease; the red blood cell information obtainer obtains the red blood cell numerical information; the quantitative value information obtainer obtains cholesterol quantitative value information representing amount of cholesterol in the urine sample; and the diagnostic support information output unit outputs at least one of the information indicating the degree of suspicion of renal disease and information related to the progression of renal disease, based on at least one of the albumin quantitative value information and the total protein quantitative value information, the cholesterol quantitative value information and the red blood cell numerical information.

Preferably, the diagnostic support information output unit further outputs urologic disease diagnostic support information related to suspected urologic disease other than renal disease based on the information obtained by the red blood cell information obtainer and the information obtained by the quantitative value information obtainer.

According to this structure, broader diagnostic support information can be output because urological disease diagnostic support information related to suspected urologic disease is output in addition to renal disease diagnostic support information related to renal disease.

Preferably, the urologic disease diagnostic support information comprises urologic disease diagnostic support information indicating that urologic disease is suspected; the red blood cell information obtainer obtains the red blood cell morphological information; and the diagnostic support information output unit outputs the urologic disease diagnostic support information indicating that urologic disease is suspected, based on the red blood cell morphological information.

According to this construction, suspected urological disease can be detected based on information obtained by the red blood cell information obtainer.

Preferably, the diagnostic support information output unit outputs the urologic disease diagnostic support information indicating that urologic disease is suspected, when the morphology of the red blood cells represented by the red blood cell morphologic information indicates an approximately uniform morphology.

Preferably, the quantitative value information obtainer obtains creatinine quantitative value information representing amount of creatinine in the urine sample; and the diagnostic support information output unit outputs the rental disease diagnostic support information based on corrected result obtained by correcting the amount of at least one of the lipid component and the protein component in the urine sample by creatinine concentration in the urine sample.

According to this construction, the precision of the renal disease diagnostic support information based on quantitative value information can be improved because the precision of the quantitative value information obtained by the quantitative value information obtainer is improved by performing creatinine correction.

Preferably, the diagnostic support information output unit comprises: a display; and a display controller for controlling the display so as to display a screen showing the renal disease diagnostic support information.

Preferably, the display controller controls the display so as to display the screen comprising a first display region for displaying analysis result of the information obtained by the red blood cell information obtainer, a second display region for displaying analysis result of the information obtained by the quantitative value information obtainer, and a third display region for displaying the renal disease diagnostic support information.

A second aspect of the present invention is a computer program product, comprising: a computer readable medium, and software instructions, on the computer readable medium, for enabling a computer to perform predetermined operations, comprising: obtaining first data comprising at least one of the number of red blood cells in a urine sample, and morphology of the red blood cells in the urine sample; obtaining second data comprising at least one of amount of protein component in the urine sample, and amount of lipid component in the urine sample; and obtaining renal disease diagnostic support information related to renal disease based on the first data and the second data.

BRIEF DESCRIPTION OF THE DRAWINGS

FIG. 1 is a perspective view showing the general structure of a urine sample analyzer using an embodiment of the diagnostic support information apparatus for renal disease of the present invention;
FIG. 2 is a block diagram showing the urine sample measurement unit of the urine sample analyzer using the diagnostic support information apparatus for renal disease of the embodiment in FIG. 1;
FIG. 3 briefly illustrates structure of the urine formed component measurement unit of the urine sample analyzer using the diagnostic support information apparatus for renal disease of the embodiment in FIG. 1;
FIG. 4 is a block diagram showing the data analysis unit of the urine sample analyzer using the diagnostic support information apparatus for renal disease of the embodiment in FIG. 1;
FIG. 5 is a block diagram showing the urine qualitative analyzer capable of sending and receiving data to/from the urine sample analyzer using the diagnostic support information apparatus for renal disease of the embodiment in FIG. 1;
FIG. 6 is a flow chart showing the operation of the urine sample analysis process performed by the urine sample analyzer using the diagnostic support information apparatus for renal disease of the embodiment in FIG. 1;
FIG. 7 is a flow chart showing the operation of the diagnostic support information obtaining process of step S16 shown in FIG. 6;
FIG. 8 shows the analysis result and diagnostic support screen of the urine sample analyzer using the diagnostic support information apparatus for renal disease of the embodiment in FIG. 1; and
FIG. 9 is a block diagram showing a modification of the diagnostic support apparatus for renal disease of the embodiment shown in FIG. 1.

DETAILED DESCRIPTION OF THE EMBODIMENT

An embodiment of the present invention is described hereinafter based on the drawings.

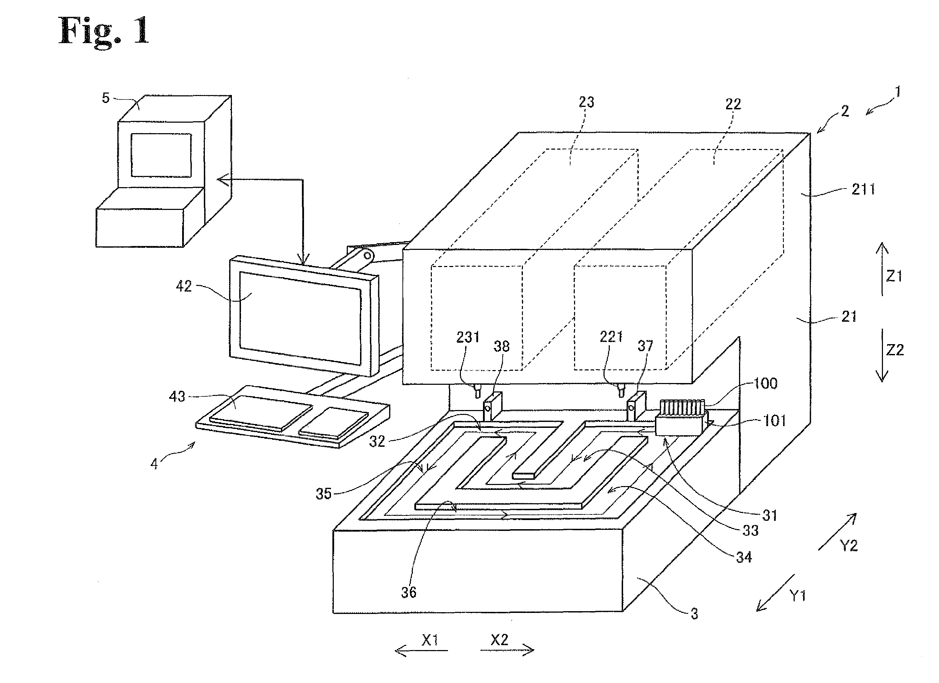

The general structure of an embodiment of the urine sample analyzer 1 of the present invention is described below with reference to FIGS. 1 through 5. Note that the present embodiment is described in terms of the diagnostic support apparatus of the present invention applied to the data analysis unit 4 of the urine sample analyzer 1.

The urine sample analyzer 1 of the present embodiment is provided with a urine sample measurement unit 2, transporting unit 3, and data analysis unit 4, as shown in FIG. 1. The data analysis unit 4 is configured to be capable of receiving a urine qualitative analysis result from an external urine qualitative analyzer 5.

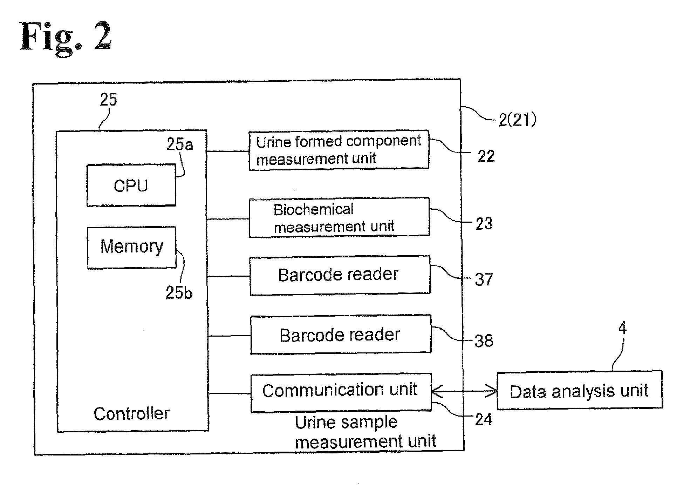

As shown in FIG. 2, the urine sample measurement unit 2 includes a main body 21, and a urine formed component measurement unit 22 and biochemical measurement unit 23, communication unit 24, and controller 25 incorporated within the single main body 21. Both the urine formed component measurement unit 22 and biochemical measurement unit 23 are covered by a unitary housing 211, as shown in FIG. 1.

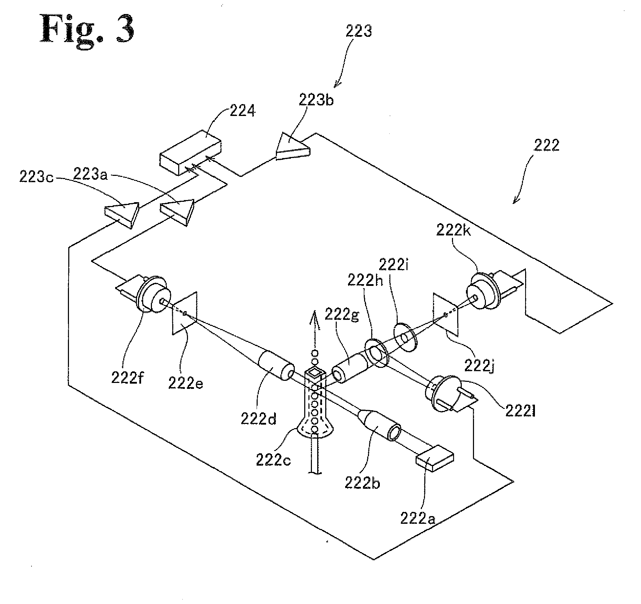

The urine formed component measurement unit 22 is configured to obtain measurement data of various measurement items related to urine formed components (red blood cells, white blood cells, epithelial cells, casts, and bacteria) by measuring a supplied urine sample via a flow cytometric method. Specifically, the measurement data obtained by the urine formed component measurement unit includes information representing the individual numbers of red blood cells, white blood cells, epithelial cells, casts, and bacteria, and morphological information representing the morphology of the red blood cells. As shown in FIG. 3, the urine formed component measurement unit 22 further includes, although not shown in the drawings, a reactor, aspirating pipette 221 (refer to FIG. 1) for aspirating a urine sample transported by the transporting unit 3, optical detection unit 222, analog signal processing circuit 223, and A/D converter 224 for converting the output of the analog signal processing circuit 223 to digital signals. The urine formed component measurement unit 22 is configured to dilute the urine sample aspirated by the aspirating pipette 221 approximately 4-fold with diluting solution and staining solution in the reactor. The reactor is configured to mix the urine sample, diluting solution, and staining solution in a staining process lasting approximately 10 seconds at a constant temperature of approximately 35°. The urine formed component measurement unit 22 also is configured to supply the urine sample prepared in the reactor, together with a sheath fluid, to a sheath flow cell 222c (refer to FIG. 3; to be described later) of the optical detection unit 222.

As shown in FIG. 3, the optical detection unit 222 includes a light emitter 222a for emitting laser light, illumination lens unit 222b, sheath flow cell 222c to be irradiated by the laser light, collecting lens 222d disposed on a line extending from the light emitter 222a in the direction of travel of the laser light, pinhole 222e and PD (photodiode) 222f, collecting lens 222g disposed in a direction intersecting the direction of travel of the laser light emitted from the light emitter 222a, dichroic mirror 222h, optical filter 222i, pinhole plate 222j provided with a pinhole and a PMT (photomultiplier tube) 222k, and PD 222l disposed at the side of the dichroic mirror 222h.

The light emitter 222a is provided to emit light on a sample flow that includes a measurement sample passing through the interior of the sheath flow cell 222c. The illumination lens unit 222b is provided to render parallel rays from the light emitted from the light emitter 222a. The PD 222f is provided to receive the forward scattered light emitted from the sheath flow cell 222c.

The dichroic mirror 222h is provided to separate the side scattered light and side fluorescent light emitted from the sheath flow cell 222c. Specifically, the dichroic mirror 222h directs the side scattered light emitted from the sheath flow cell 222c to the PD 222l, and directs the side fluorescent light emitted from the sheath flow cell 222c to the PMT 222k. The PD 222l is provided to receive the side scattered light. The PMT 222k is provided to receive the side fluorescent light. The PDs 222f and 222l, and the PMT 222k respectively have the function of converting the received optical signals to electrical signals.

The analog signal processing circuit 223 includes amps 223a, 223b, and 223c, as shown in FIG. 3. The amps 223a, 223b, and 223c are respectively provided to perform amplification and waveform processing on the electrical signals output from the PD 222f, PMT 222k, and PD 222l. The amplified and waveform processed electrical signals are output to the controller 25 through the A/D converter 224.

The biochemical measurement unit 23 has the function of obtaining quantitative information representing the amounts of the biochemical components contained in the urine sample. Specifically, the biochemical measurement unit 23 is configured to accept the urine sample into the apparatus, and dispense reagents to the accepted urine sample. The biochemical measurement unit 23 also is configured to measure the transmission light produced by the light illuminating the urine sample that contains the dispensed reagent. Thus, the biochemical measurement unit 23 is configured to obtain quantitative information relating to the biochemical components contained in the urine sample by measuring the urine sample using optical methods. Specifically, the biochemical measurement unit 23 obtains biochemical quantitative measurement data representing the amounts of total protein, albumin, cholesterol, and creatinine in the urine sample. The biochemical measurement unit 23 is further configured to aspirate the urine sample transported by the transporting unit 3 via the aspirating pipette 231 (refer to FIG. 1), and accept the sample into the measurement unit.

The communication unit 24 is an Ethernet (registered trademark) interface, and the urine sample measurement unit 2 is connected to the data analysis unit 4 through the communication unit 24 via a LAN cable. The urine sample measurement unit 2 is configured to transmit, to the data analysis unit 4, the various measurement data obtained from the urine formed component measurement unit 22 and the biochemical measurement unit 23 using a predetermined communication protocol (TCP/IP).

The controller 25 has a CPU 25a and a memory 25b. The CPU 25a is configured to control the operations of each unit by executing computer programs stored in the memory 25b. That is, the CPU 25a is configured to control the operations of both the urine formed component measurement unit 22 and the biochemical measurement unit 23.

The transporting unit 3 is disposed on the front side (side in the arrow Y1 direction) of the urine sample measurement unit 2, as shown in FIG. 3. The transporting unit 3 is configured to transport the urine sample to the urine formed component measurement unit 22 and the biochemical measurement unit 23 of the urine sample measuring unit 2. Specifically, the transporting unit 3 is configured to transport a sample container 100 held in a rack 101 to a position (urine sample obtaining position of the urine formed component measurement unit 22) below the aspirating pipette 221 of the urine formed component measurement unit 22 and to a position (urine sample obtaining position of the biochemical measurement unit 23) below the aspirating pipette 231 of the biochemical measurement unit 23 by moving the rack 101.

The transporting unit 3 is configured to circulate a rack 101 capable of holding a plurality of sample containers 100 through a urine sample obtaining position of the urine formed component measurement unit 22, and a urine sample obtaining position of the biochemical measurement unit 23. Specifically, the transporting unit 3 has a first transverse unit 31, second transverse unit 32, and a buffer unit 33 disposed between the first transverse unit 31 and the second transverse unit 32. The transporting unit 3 also has a rack feeder 34 for feeding the rack 101 to the first transverse unit 31, rack take-up 35 for taking up the rack 101 from the second transverse unit 32, and a third transverse unit 36 disposed to the front of the buffer unit 33 and connected to the rack feeder 34 and rack take-up 35. That is, the transporting unit 3 has a circulation transport path configured by the first transverse unit 31, buffer unit 33, second transverse unit 32, rack feeder 35, and third transverse unit 36. Thus, the transporting unit 3 can deposit the sample container 100 at the urine sample obtaining position of the urine formed component measurement unit 22 and the urine sample obtaining position of the biochemical measurement unit 23 while the rack 101 is circulating along the circulation transport path.

The transporting unit 3 is also provided with barcode readers 37 and 38 for reading the barcodes (not shown in the drawings) adhered to each sample container 100. Specifically, the barcode reader 37 is disposed along the first transverse unit 31 on the rack feeder 34 side (side in the arrow X2 direction) from the urine sample obtaining position of the urine formed component measurement unit 22. Therefore, the barcode reader 37 reads the barcode of the sample container 100 transported by the first transverse unit 31 before the urine sample is aspirated by the aspirating pipette 221. The barcode reader 38 is disposed along the second transverse unit 32 on the buffer 33 side (side in the arrow X2 direction) from the urine sample obtaining position of the biochemical measurement unit 23. Thus, the barcode reader 38 can read the barcode of the sample container 100 transported by the second transverse unit 32 before the sample is aspirated by the aspirating pipette 231. The barcode readers 37 and 38 are respectively configured to transmit the read sample ID (barcode information) to the data analysis unit 4 whenever required. Note that the barcode adhered to each sample container 100 is unique for each individual urine sample and is used to manage the urine samples.

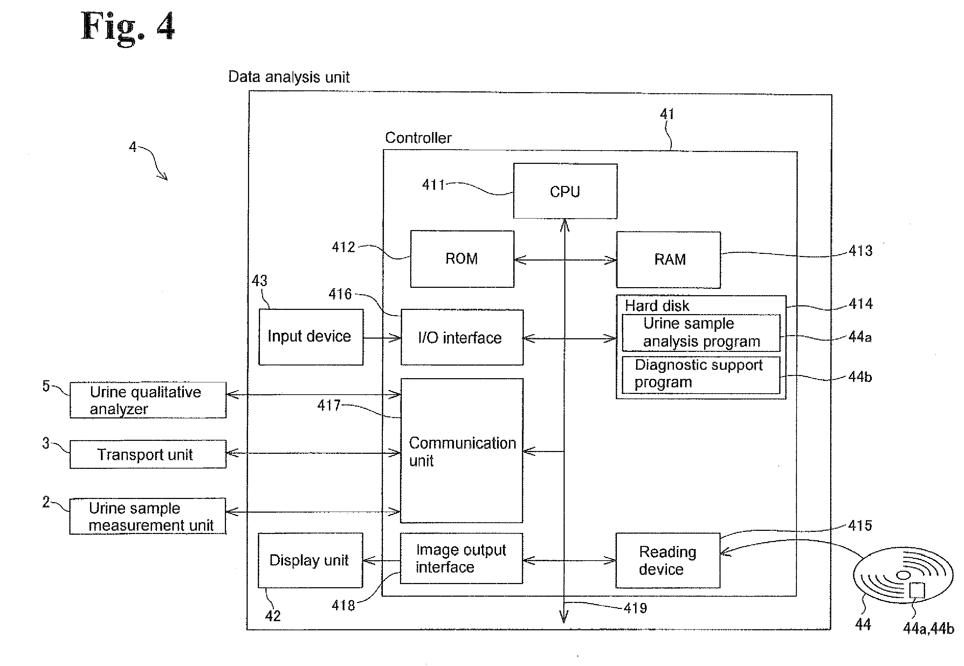

As shown in FIGS. 1 and 4, the data analysis unit 4 is a computer mainly configured by a controller 41 (refer to FIG. 4), display unit 42, and input device 43.

As shown in FIG. 4, the controller 41 is mainly configured by a CPU 411, ROM 412, RAM 413, hard disk 414, reading device 415, I/O (input/output) interface 416, communication unit 417, and image output interface 418. The CPU 411, ROM 412, RAM 413, hard disk 414, reading device 415, I/O interface 416, communication interface 417, and image output interface 418 are mutually connected via a bus 419.

The CPU 411 executes the computer programs stored in the ROM 412 and the computer programs loaded in the RAM 413. The computer functions as the data analysis unit 4 when the CPU 411 executes the urine sample analysis program 44a and diagnostic support program 44b which will be described later.

In the present embodiment, the CPU 411 of the data analysis unit 4 obtains a urine formed component analysis result by analyzing the measurement data obtained by the urine formed component measurement unit 22 and received via the communication unit 417. Specifically, the CPU 411 is configured to obtain concentration information of each type cell and red blood cell particle distribution information representing the morphology of the red blood cells by analyzing the urine formed component measurement data of the red blood cells (RBC), white blood cells (WBC), epithelial cells (EP), urinary casts (CAST), And bacteria (BACT). The CPU 411 is further configured to obtain quantitative biochemical analysis results by analyzing the measurement data obtained by the biochemical measurement unit 23 and received through the communication unit 417. Specifically, the CPU 411 is configured to obtain the micro total protein concentration representing the concentration of the total protein (micro total protein) in the urine sample, micro albumin concentration representing the albumin concentration (micro albumin) in the urine sample, micro cholesterol concentration representing the cholesterol concentration (micro cholesterol) in the urine sample, and micro creatinine concentration representing the creatinine concentration (micro creatinine) in the urine sample. The CPU 411 jointly analyzes both the measurement data obtained by the urine formed component measurement unit 22 and the measurement data obtained by the biochemical measurement unit 23.

The CPU 411 also obtains excretion estimate values per day for micro total protein, micro albumin, and micro cholesterol by performing creatinine correction using the obtained micro creatinine concentration.

Specifically, the CPU 411 obtains the micro total protein estimated excretion value (m-TP) per day, micro albumin estimated excretion value (m-ALB) per day, and micro cholesterol estimated excretion value (m-CHO) per day based on equation (1) below.

$$Z = X \times a \div Y \cdot \cdot \cdot \cdot \cdot (1)$$

In equation (1), Z represents the estimated excretion value (mg) per day of the target component, X represents the concentration (mg/dL) of the target component, Y represents the micro creatinine concentration (g/dL), and a represents a coefficient derived from the body type, age, and sex of the subject. Note that in equation (1), the amount of creatinine excretion in urine per day by a subject with standard muscle mass is assumed to be 1 g. The creatinine concentration is known to be proportional to the muscle mass of the subject, so that it is desirable that a>1 when the subject is, for example, a young male adult with excess muscle mass, and a<1 when the subject is an older female with less muscle mass.

The ROM 412 is configured by a mask ROM, PROM, EPROM, EEPROM or the like, and stores the computer programs to be executed by the CPU 411 as well as the data used by these computer programs.

The RAM 413 is configured by SRAM, DRAM or the like. The RAM 413 is used when reading the computer programs stored in the ROM 412 and the hard disk 414. The RAM 413 is also used as the work area of the CPU 411 when the CPU 411 executes these computer programs.

The hard disk 414 stores an operating system, application programs and the like, and the various computer programs to be executed by the CPU 411 as well as the data used in the execution of the computer programs. The urine sample analysis program 44a and diagnostic support program 44b are installed on the hard disk 414.

The reading device 415 is configured by a floppy disk drive, CD-ROM drive, DVD-ROM drive or the like, and is capable of reading computer programs or data recorded on a portable recording medium 44. The portable recording medium 444 stores the urine sample analysis program 44a and the diagnostic support program 44b, and the computer can read the urine sample analysis program 44a and the diagnostic support program 44b from the portable recording medium 44 and install the urine sample analysis program 44a and the diagnostic support program 44b on the hard disk 414.

Note that the urine sample analysis program 44a and the diagnostic support program 44b can not only be provided by the portable recording medium 44, the urine sample analysis program 44a and the diagnostic support program 44b may also be provided over an electrical communication line from an external device which is connected to the computer via the electrical communication line (either wireless or wired) so as to be capable of communication. For example, the urine sample analysis program 44a and the diagnostic support program 44b may be stored on the hard disk of a server computer on the Internet so that the computer can access the server computer, download the urine sample analysis program 44a and the diagnostic support program 44b, and install the urine sample analysis program 44a and the diagnostic support program 44b on the hard disk 414.

An operating system that provides a graphical user interface environment, such as, for example, Microsoft Windows (registered trademark of Microsoft Corporation, USA), is also installed on the hard disk 414. In the following description, the urine sample analysis program 44a and the diagnostic support program 44b also operate on this operating system.

The I/O interface 416 is configured by a serial interface such as a USB, IEEE1394, RS232C or the like, parallel interface such as SCSI, IDE, IEEE1284 or the like, analog interface such as a D/A converter, A/D converter or the like. The I/O interface 416 is connected to the input device 43 so that a user can input data into the computer using the input device 43. Thus, the user can issue, to the data analysis unit 4, a measurement instruction (input a measurement order) for the urine formed component measurement unit 22 and issue a measurement instruction (input a measurement order) for the biochemical measurement unit 23.

The communication unit 417 is, for example, an Ethernet (registered trademark) interface. The data analysis unit 4 is configured to receive measurement data through the communication unit 417 from the urine formed component measurement unit 22 and biochemical measurement unit 23. The data analysis unit 4 is further configured to receive, through the communication unit 417, urine qualitative analysis result information from the external urine qualitative analyzer 5. The data analysis unit 4 also transmits controls signals through the communication unit 417 to the transport unit 3 and urine sample measurement unit 2.

The image output interface 418 is connected to the display unit 42 which is configured by an LCD, CRT or the like, and outputs image signals corresponding to the image data from the CPU 411 to the display unit 42. The display unit 42 displays images (screens) according to the input image signals.

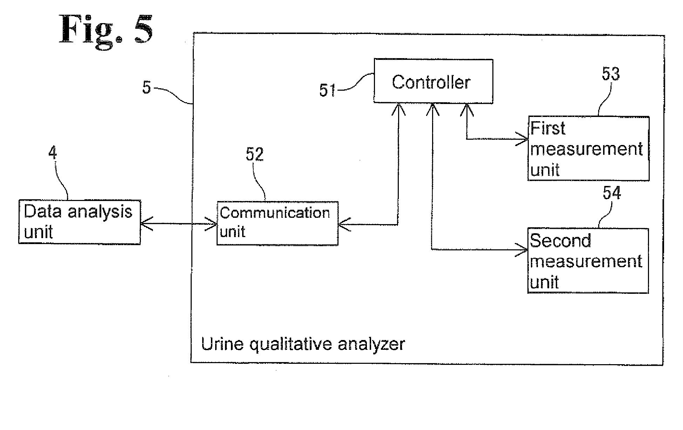

As shown in FIG. 5, the urine qualitative analyzer 5 has controller 51 configured by a CPU, ROM, RAM and the like, and further has a communication unit 52 for sending and receiving data to/from the data analysis unit 4, and first measurement unit 53 and second measurement unit 54 for performing measurements of the urine sample. The urine qualitative analyzer 5 has the function of performing qualitative evaluations of occult blood concentration, protein concentration, white blood cell concentration, nitrite concentration, and glucose concentration.

The controller 51 classifies the occult blood concentration, protein concentration, white blood cell concentration, nitrite concentration, and glucose concentration measured by the first measurement unit 53 (described later) in nine stages, that is, (-), ($\pm$), (+), (2+), (3+), (4+), (5+), (6+), (7+). The controller 51 classifies the urine sample in these none stages based on the degree of color change of a litmus paper used by the first measurement unit 53. The controller 51 sends this classification result (analysis result) information and the specific gravity information obtained by the second measurement unit 54 through the communication unit 52 to the data analysis unit 4.

The first measurement unit 53 is configured to measure the occult blood concentration, protein concentration, white blood cell concentration, nitrite concentration, and glucose concentration of the urine sample. The second measurement unit 54 is configured to measure the specific gravity of the urine sample.

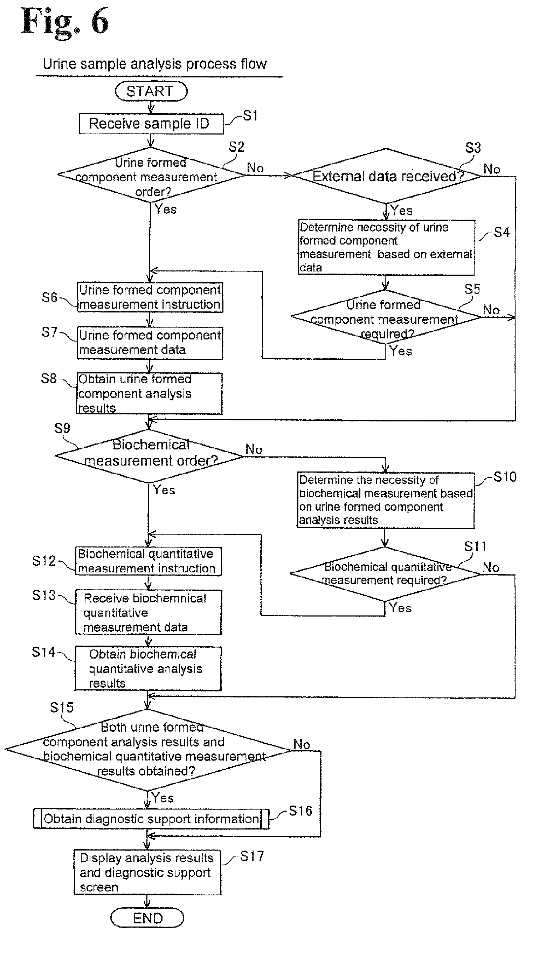

The operation of the urine sample analysis process performed by the urine sample analysis program 44a of the present embodiment of the urine analyzer 1 is described below with reference to FIG. 6.

In step S1, the CPU 411 receives, the urine sample ID (barcode information) of the measurement target from the barcode reader 37. In step S2, the CPU 411 determines whether there is a measurement instruction (measurement order) for the urine formed component measurement unit 22 from the user, and proceeds to step S6 when there is a measurement instruction. When there is not a measurement instruction, however, the CPU 411 determines in step S3 whether external data have been received, and proceeds to step S9 when not external data have been received. The external data are data obtained from the external urine qualitative analyzer 5. When external data have been received, the CPU 411 determines in step S4 whether a measurement must be performed by the urine formed component measurement unit 22 based on the external data. Specifically, for example, when urine qualitative analysis result information has been received from the urine qualitative analyzer 5, the CPU 411 determines whether the urine formed component measurement unit 22 must perform a measurement based on the degree of occult blood concentration classified in nine stages. The process continues to step S6 if the urine formed component measurement unit 22 must perform a measurement in step S5, whereas the process advances to step S9 if the urine formed component measurement unit 22 is not required to perform a measurement.

In step S6, the CPU 411 issues a measurement instruction to the urine formed component measurement unit 22. Specifically, the controller 25 of the urine sample measurement unit 2 received, through the communication unit 24, a measurement instruction signal sent by the CPU 411 through the communication unit 417, and the controller 25 starts the measurement operation of the urine formed component measurement unit 22. Thus, the urine formed measurement component measurement unit 22 aspirates the urine sample measurement target from the sample container 100 disposed at the urine sample obtaining position, and obtains the measurement data relating to the red blood cells (RBC), white blood cells (WBC), epithelial cells (EC), urinary casts (CAST), and bacteria (BACT) in the urine sample. The controller 52 transmits the urine formed component measurement data obtained by the urine formed component measurement unit 22 to the data analysis unit 4 through the communication unit 24. Thereafter, in step S7, the CPU 411 receives the measurement data relating to the red blood cells (RBC), white blood cells (WBC), epithelial cells (EC), urinary casts (CAST), and bacteria (BACT) in the urine sample from the urine formed component measurement unit 22. In step S8, the CPU 411 then analyzes the received urine formed component measurement data. The red blood cell particle distribution information representing the morphological information of the red blood cells and the concentration information of each type of cell, that is, red blood cells (RBC), white blood cells (WBC), epithelial cells (EC), urinary casts (CAST), and bacteria (BACT) are then obtained as the urine formed component analysis result.

In step S9, the CPU 411 determines whether there is a measurement instruction (measurement order) for the biochemical measurement unit 23 from the user, and proceeds to step S12 when there is a measurement instruction. When there is no measurement instruction, the CPU 411 determines in step S10 whether a measurement is required by the biochemical measurement unit 23 based on the urine formed component analysis result. Specifically, if the red blood cell concentration is below a predetermined threshold value, the CPU 411 determines that measurement is required by the biochemical measurement unit 23, whereas the CPU 411 determines that measurement is not required by the biochemical measurement unit 23 when the red blood cell concentration is equal to or above the predetermined threshold value. In this case, whether a measurement is required by the biochemical measurement unit 23 may be determined based on the concentration information of a cell other than the red blood cells, or whether a measurement is required by the biochemical measurement unit 23 may be determined based on the concentration information of a plurality of types of cells. Whether a measurement is required by the biochemical measurement unit 23 may also be determined based on the red blood cell particle distribution information. The process moves to step S12 when the CPU 411 has determined that measurement is required by the biochemical measurement unit 23 in step S11, whereas the process advances to step S15 when the CPU 411 has determined that measurement by the biochemical measurement unit 23 is unnecessary.

In step S12, the CPU 411 issues a measurement instruction to the biochemical measurement unit 23. Specifically, the controller 25 of the urine sample measurement unit 2 received, through the communication unit 24, a measurement instruction signal sent by the CPU 411 through the communication unit 417, and the controller 25 starts the measurement operation of the biochemical measurement unit 23. Thus, the biochemical measurement unit 23 aspirates the measurement target urine sample from the sample container 100 disposed at the urine sample obtaining position, and obtains the biochemical quantitative measurement data of the total protein, albumin, cholesterol, and creatinine in the urine sample. The controller 52 transmits the biochemical quantitative measurement data obtained by the biochemical measurement unit 23 to the data analysis unit 4 through the communication unit 24. Thereafter, in step S13, the CPU 411 receives the biochemical quantitative measurement data of the total protein, albumin, cholesterol, and creatinine in the urine sample from the biochemical measurement unit 23. In step S14, the CPU 411 analyzes the received biochemical quantitative measurement data. Thus, the micro total protein concentration, micro albumin concentration, micro cholesterol concentration, and micro creatinine concentration are received as biochemical quantitative analysis results. The micro total protein estimated excretion value (m-TP) per day, micro albumin estimated excretion value (m-ALB) per day, and micro cholesterol estimated excretion value (m-CHO) per day are then obtained as biochemical quantitative analysis results by correcting the creatinine value using the micro creatinine concentration.

In step S15, the CPU 411 determines whether both the urine formed component analysis results and the biochemical quantitative analysis results have been obtained, and advances to step S17 when either one of the two analysis results is missing. When both analysis results have been obtained, the CPU 411 executes the operation of the diagnostic support information obtaining process in step S16.

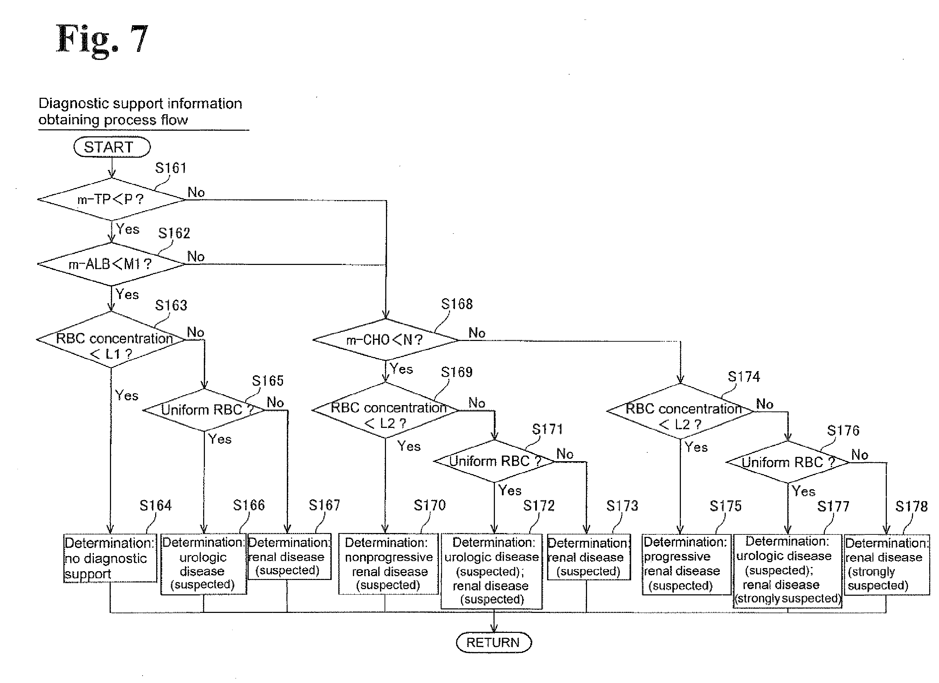

The diagnostic support information obtaining process performed by the diagnostic support program 44b in step S16 of FIG. 6 is described below with reference to FIG. 7.

In step S161, the CPU 411 determines whether the micro total protein estimated excretion value (m-TP) obtained as a biochemical quantitative analysis result is less than the threshold value P, and the process advances to step S162 when the estimated value is less than the threshold value P. In step S162, the CPU 411 determines whether the micro albumin estimated excretion value (m-ALB) obtained as a biochemical quantitative analysis result is less than the threshold value M, and the process advances to step S163 when the estimated value is less than the threshold value M.

In step S163, the CPU 411 determines whether the red blood cell concentration obtained as a urine formed component analysis result is less than a predetermined threshold value L1, and when the RBC concentration is less than the threshold value L1, the CPU 411 determines in step S164 there is no diagnostic support information. That is, the CPU 411 determines that there is no diagnostic support information when all conditions (1) through (3) are satisfied relative to the target urine sample, the conditions being: (1) the micro total protein estimated excretion value (m-TP) is less than the threshold value P, (2) the micro albumin estimated excretion value (m-ALB) is less than the threshold value M1, and (3) the red blood cell concentration is less than the predetermined threshold value L1.

On the other hand, when the red blood cell concentration is equal to or greater than the predetermined threshold value L1 in step S163, the CPU 411 determines in step S165 whether the red blood cells in the urine sample are uniform red blood cells. Specifically, the CPU 411 determines whether the majority of the red blood cells in the urine sample are uniform red blood cells with a low degree of morphological deformation (small amount deformation) or the majority of red blood cells are nonuniform red blood cells with a high degree of morphological deformation (large amount of deformation). In the case of uniform red blood cells, the CPU 411 determines in step S166 that there is a possibility of urologic disease, whereas the CPU 411 determines in step S167 that there is a possibility of renal disease in the case of nonuniform red blood cells. Note that whether red blood cells have been present in the urine over a long period can be estimated by determining whether the red blood cells are uniform, and there is concern of renal disease when red blood cells have been present in the urine over a long period, whereas there is at least a possibility of urologic disease when red blood cells have been present in the urine over a short time.

That is, it is possible to determine there is possibility of suspected renal disease since there is a high degree of morphological deformation and nonuniform red blood cells when red blood cells have been present in the urine over a long period, and it is possible to determine there is at least a possibility of suspected urologic disease since there is a low degree of morphological deformation and uniform red blood cells when red blood cells have been present in the urine over a short period. Note that it may also be determined that there is a suspicion of renal disease in addition to urologic disease even when red blood cells have not been present in the urine over a short period and there is a low degree of morphological deformation. In the present embodiment, diagnostic support information relating to the suspicion of urologic disease as well as renal disease is obtained, and that the suspicion of renal disease and the suspicion of urologic disease are determined based on the red blood cell morphological information.

When the micro total protein estimated excretion value (m-TP) is equal to or greater than the threshold value P, or when the micro albumin estimated excretion value (m-ALB) is equal to or greater than the threshold value M1, the CPU 411 determines in step S168 whether the micro cholesterol estimated excretion value (m-CHO) obtained as a biochemical quantitative analysis result is less than a threshold value N. When the micro cholesterol estimated excretion value (m-CHO) is less than the threshold value N, the CPU 411 determines in step S169 whether the red blood cell concentration obtained as a urine formed component analysis result is less than the predetermined threshold value L2, and when the RBC concentration is less than the threshold value L2, the CPU 411 determines in step S170 that there is suspected nonprogressive renal disease.

When the red blood cell concentration is equal to or greater than the threshold value L2 in step S169, the CPU 411 determines in step S171 whether the red blood cells in the urine sample are uniform red blood cells, and when the red blood cells are uniform cells, the CPU 411 determines in step S172 that there is suspected urological disease and suspected renal disease. When the cells are not uniform red blood cells, the CPU 411 determines in step S173 that there is suspected renal disease. Note that the predetermined concentration L1 may be identical to the predetermined concentration L2, although it is preferred that the predetermined concentration L1 is less than the predetermined concentration L2.

When the micro cholesterol estimated excretion value (m-CHO) is equal to or greater than the threshold value N in step S168, the CPU 411 determines in step S174 whether the red blood cell concentration obtained as a urine formed component analysis result is less than the predetermined value L2, and when the RBC concentration is less than the threshold value L2, the CPU 411 determines in step S175 that there is suspected progressive renal disease.

When the red blood cell concentration is equal to or greater than the threshold value L2 in step S174, the CPU 411 determines in step S176 whether the red blood cells in the urine sample are uniform red blood cells, and when the red blood cells are uniform cells, the CPU 411 determines in step S177 that there is suspected urological disease and strongly suspected renal disease. When the cells are not uniform red blood cells, the CPU 411 determines in step S178 that there is a strong suspicion of renal disease.

In the embodiment described above, when the micro total protein estimated excretion value (m-TP) is less than the threshold value P or the micro albumin estimated excretion value (m-ALB) is less than the threshold value M1, a determination of suspected renal disease (including strong suspicion) is obtained regardless of the micro cholesterol estimated excretion value (m-CHO) and whether the RBC concentration value indicates uniform red blood cells.

When the micro total protein estimated excretion value (m-TP) is less than the threshold value P and the micro cholesterol estimated excretion value (m-CHO) is also less than the threshold value N, or the micro albumin estimated excretion value (m-ALB) is less than the threshold M1 and the micro cholesterol estimated excretion value (m-CHO) is also less than the threshold value N, suspected urological disease and strongly suspected renal disease are determined regardless of the red blood cell concentration value and whether the RBC are uniform. That is, the degree of renal disease is determined based on the micro cholesterol estimated excretion value (m-CHO). When the micro total protein estimated excretion value (m-TP) is not less than the threshold value P and the micro albumin estimated excretion value (m-ALB) is not less than the threshold value M1, or the micro cholesterol estimated excretion value (m-CHO) is equal to or greater than the threshold value N, a determination of progressive renal disease is obtained regardless of the red blood cell concentration and whether the RBC are uniform cells. In the present embodiment, progressive renal disease is thus determined based on the micro cholesterol estimated excretion value (m-CHO).

Note that in the present embodiment diagnostic support information can be obtained relating to suspected renal disease indicated by abnormal values in the biochemical quantitative analysis, for example, IgA nephropathy, acute progressive nephritis, diabetes mellitus and the like, by performing a biochemical component quantitative examination in conjunction with a urine formed component examination as described above. Diagnostic support information can also be obtained relating to suspected diseases such as cystitis, urethritis and the like as urological disease not involving the renals.

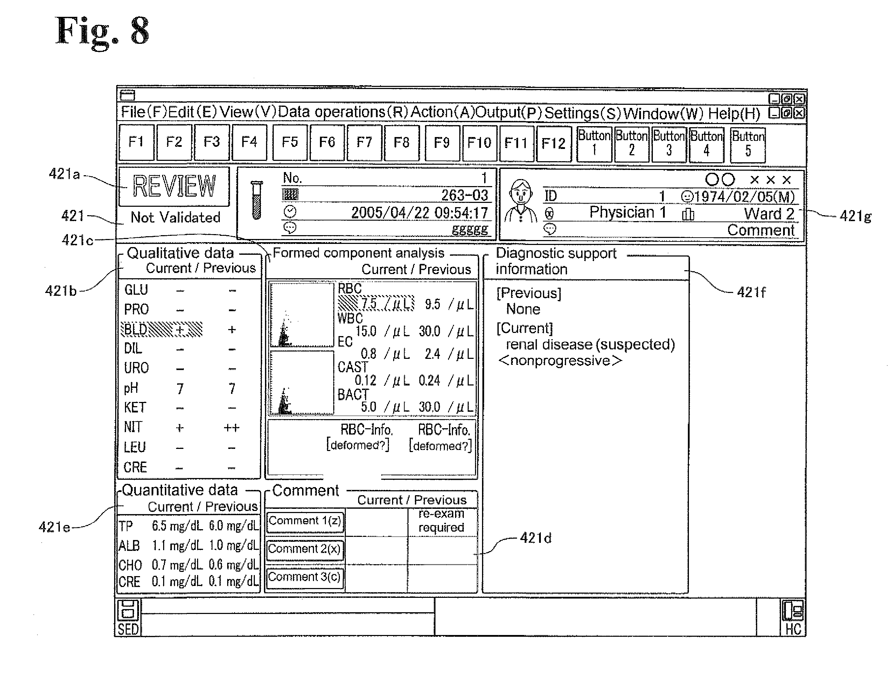

After the diagnostic support information obtaining process of step S16, the CPU 411 displays the analysis result and diagnostic support screen 421 on the display unit 42 in step S17 of FIG. 6, and the operation of the urine sample analysis process ends, as shown in FIG. 8. When re-analysis of the corresponding urine sample is desired, the REVIEW display 421 a is displayed in a red-colored highlight on the analysis result and diagnostic support screen 421. The analysis result and diagnostic support screen 421 also has a qualitative data region 421b for displaying urine qualitative analysis results received from the external qualitative urine analyzer 5, a formed component analysis results region 421 c for displaying the urine formed component analysis results, and a comment field 421 d. The analysis result and diagnostic support screen 421 further has a quantitative data region 421e for displaying biochemical quantitative analysis results, diagnostic support information region 421f for displaying diagnostic support information, and subject information region 421 g for displaying subject information.

The urine qualitative analysis results for both the previous and current results of each component are displayed in the qualitative data region 421 b. The formed component analysis region 421c displays the concentration values for both the previous and current results of each cell, that is, red blood cells (RBC), white blood cells (WBC), epithelial cells (EC), casts (CAST), and bacteria (BACT). The formed component analysis region 421c also displays two-dimensional scattergrams plotting the particles in the urine sample based on the scattered light intensity and fluorescent light intensity.

The quantitative data region 421 e displays the concentration values for both the previous and current results of each component, that is, micro total protein concentration (TP), micro albumin concentration (ALB), micro cholesterol concentration (CHO), and micro creatinine concentration (CRE). Note that the quantitative data region 421 e can also display the micro total protein estimated excretion value (m-TP) per day, micro albumin estimated excretion value (m-ALB) per day, and micro cholesterol estimated excretion value (m-CHO) per day by changing the display setting. The diagnostic support information region 421f displays both the previous diagnostic support information and the current diagnostic support information obtained by the diagnostic support information obtaining process shown in FIG. 7. The patient information region 421g displays the patient identification information which includes the patient name, birth date and the like.

In the present embodiment described above, the CPU 41 of the data analysis unit 4 obtains diagnostic support information related to suspected renal disease based on the concentration information of red blood cells in the urine sample and red blood cell particle distribution information representing the red blood cell morphology, micro total protein estimated excretion value (m-TP) per day based on the total protein in the urine sample, micro albumin estimated excretion value (m-ALB) per day based on the amount of albumin in the urine sample, and micro cholesterol estimated excretion value (m-CHO) per day based on the amount of cholesterol in the urine sample, and outputs the diagnostic support information related to suspected renal disease to the diagnostic support information region 421f in the analysis result and diagnostic support screen 421 of the display unit 42. Thus, suspected renal disease can be detected using the output diagnostic support information even without a specialist physician. Suspected renal disease can therefore be detected at an early stage since suspected renal disease can be detected by a nonspecialist physician if the data analysis unit 4 is used as the diagnostic support apparatus for renal disease of the present invention even in the stages prior to the confirming diagnosis by a specialist physician in the diagnostic process for renal disease performed over several stages.

In the present embodiment, the CPU 411 of the data analysis unit 4 determines there is at least suspected renal disease when at least one of the micro albumin estimated excretion value (m-ALB) is equal to or greater than the threshold value M1, and the micro total protein estimated excretion value (m-TP) is equal to or greater than the threshold value P. In this way at least suspected renal disease can be uniformly determined based on the magnitude relationship between the micro albumin estimated excretion value (m-ALB) and the threshold value M1, and the magnitude relationship between the micro total protein estimated excretion value (m-TP) and the threshold value (m-TP).

In the present embodiment, the CPU 411 of the data analysis unit 4 determines the degree (suspected or strongly suspected) of suspected renal disease and the whether the renal disease is progressive (progressive or nonprogressive) based on the micro cholesterol estimated excretion value (m-CHO) when at least one of the micro albumin estimated excretion value (m-ALB) is equal to or greater than the threshold value M1 or the micro total protein estimated excretion value (m-TP) is equal to or greater than the threshold value P. Therefore, the degree of suspected renal disease and the progressivity of the renal disease can be determined in addition to the presence of suspected renal disease.

In the present embodiment, the CPU 411 of the data analysis unit 4 determines suspected renal disease and suspected urological disease (or both suspected renal disease and urological disease) from the red blood cell particle distribution information according to whether the degree of morphological deformation is slight (small amount of deformation) or the degree of morphological deformation is large (large amount of deformation). Therefore, it is possible to provide at least one of diagnostic support information related to renal disease and diagnostic support information related to urological disease based on the red blood cell particle distribution information.

In the present embodiment, the CPU 411 of the data analysis unit 4 obtains the estimated excretion amounts per day of the total protein, albumin, and cholesterol based on the amounts of total protein, albumin, and cholesterol in the urine sample by using the creatinine concentration to perform creatinine correction on the total protein, albumin, and cholesterol in the urine sample. Therefore, the precision of the diagnostic support information based on the quantitative values is improved because the precision of the quantitative values (estimated amount of excretion per day) is improved based on the amounts of the components in the urine sample.

Note that, in the embodiment of this disclosure, all aspects are examples and should not be considered as limiting. The scope of the present invention is defined by the scope of the claims and not be the description of the embodiment, and includes all modifications within the scope of the claims and the meanings and equivalences therein.

For example, the embodiment is described by way of example in which the data analysis unit obtains red blood cell distribution information and concentration information of red blood cells, white blood cells, epithelial cells, casts, and bacteria, the present invention is not limited to this example inasmuch as either one of the red blood cell concentration in the urine or the red blood cell particle distribution information may be obtained. In this case, the red blood cell concentration may be replaced by the red blood cell count insofar as the red blood cell count value is based on the number of red blood cells in the urine sample.

Although in the above embodiment the urine formed component measurement unit measures red blood cells by a flow cytometric method, and the data analysis unit obtains red blood cell particle distribution information representing the morphology of the red blood cells based on the measurement information obtained by the urine formed component measurement unit, the present invention is not limited to this arrangement. For example, the red blood cell morphology (information representing whether the red blood cells are uniform or nonuniform) can be determined by the urine formed component measurement unit imaging the red blood cells in the urine sample, and the data analysis unit performing image processing on the red blood cell images obtained by the urine formed component measurement unit.

Although concentration information of the red blood cells, white blood cells, epithelial cells, casts, and bacteria in the urine sample are obtained by the data analysis unit in the above embodiment, the present invention is not limited to this arrangement. The concentration information of the red blood cells, white blood cells, epithelial cells, casts, and bacteria in the urine sample need not be obtained insofar as the concentration information of red blood cells in the urine sample can be obtained.

Although the micro total protein concentration, micro albumin concentration, micro cholesterol concentration, and micro creatinine concentration are obtained in the above embodiment, the present invention is not limited to this arrangement. The other component concentrations need not be obtained insofar as at least one of the micro total protein concentration, micro albumin concentration, micro cholesterol concentration can be obtained.

Although the data analysis unit provides diagnostic support information related to renal disease based on the red blood cell concentration information and red blood cell particle distribution information, and micro total protein estimated excretion value (m-TP), micro albumin estimated excretion value (m-ALB), and micro cholesterol estimated excretion value (m-CHO) in the above embodiment, the present invention is not limited to this arrangement. Diagnostic support information may be provided based on at least one of the red blood cell concentration information and red blood cell particle distribution information, and at least one of the micro total protein estimated excretion value (m-TP), micro albumin estimated excretion value (m-ALB), and micro cholesterol estimated excretion value (m-CHO), and diagnostic support information also may be provided based on the information of the red blood cell concentration information and particle distribution information, and at least one of the estimated excretion values of the other components.

Although the data analysis unit provides diagnostic support information related to renal disease and diagnostic support information related to urological disease in the above embodiment, the present invention is not limited to this arrangement. The data analysis unit also may provide only diagnostic support information related to renal disease.

Although the above embodiment is described by way of example in which the presence of suspected renal disease and the degree of suspected renal disease are determined by the data analysis unit determining [renal disease (suspected)] or [renal disease (strongly suspected)], the present invention is not limited to this example. The degree of suspected renal disease also may be provided, for example, by displaying the degree of suspected renal disease as a numeric value from 1 to 10. The presence of suspected renal disease also may be provided, for example, by displaying the specific disease name such as [IgA nephropathy].

Although the above embodiment has been described by way of example in which the data analysis unit obtains information related to the presence of suspected renal disease, and the degree of suspected renal disease, and progressivity of renal disease as diagnostic support information related to suspected renal disease, the present invention is not limited to this example. Other information related to suspected renal disease also may be obtained and output as diagnostic support information.

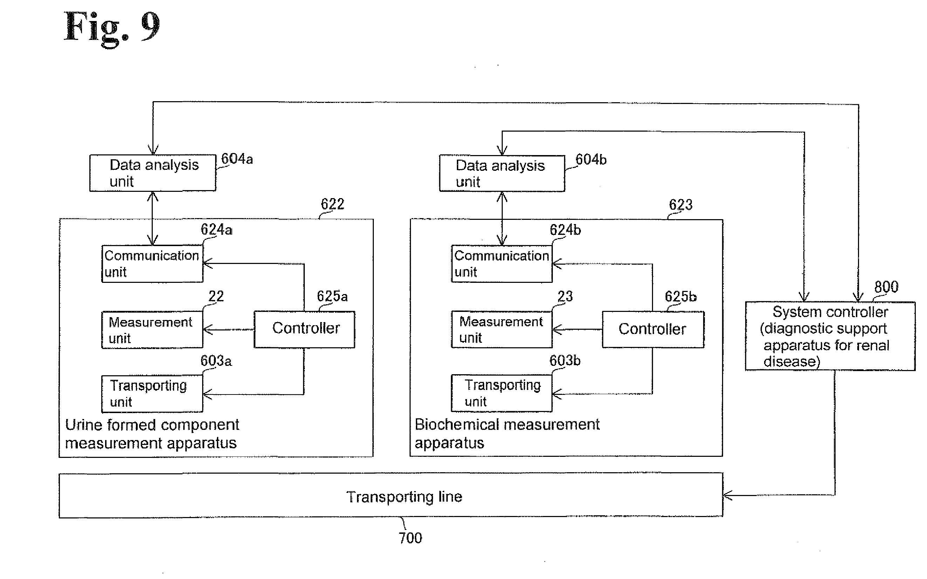

Although the above embodiment has been described by way of example in which the urine formed component measurement unit and the biochemical measurement unit are housed within a single apparatus, the present invention is not limited to this example. A urine formed component measurement apparatus and the biochemical measurement apparatus also may be provided separately and provided with respective transporting units as shown in the modification shown in FIG. 9. In this modification, a system controller 800, which is an example of the diagnostic support apparatus for renal disease, is connected to a urine formed component measurement apparatus 622 and a biochemical measurement apparatus 623 through data analysis units 604a and 604b, which are configured by computers. The system controller 800 is connected to a transport line 700 for transporting urine samples to the urine formed component measurement apparatus 622 and the biochemical measurement apparatus 623, and has the functions of a host computer for controlling each apparatus, The urine formed component measurement apparatus 622 (biochemical measurement unit 623) also has a measurement unit 22 (23), communication unit 624a (624b), transporting unit 603a (603b), and controller 625a (625b). The measurement data obtained by the urine formed component measurement apparatus 622 is analyzed by the data analysis unit 604a and then transmitted to the system controller 800. The measurement data obtained by the biochemical measurement apparatus 623 is analyzed by the data analysis unit 604b and then transmitted to the system controller 800. The system controller 800 obtains and outputs diagnostic support information based on the urine formed component analysis result and the biochemical quantitative analysis result obtained from the data analysis units 604a and 604b. Thus, the diagnostic support apparatus for renal disease also is applicable to a system controller (host computer) of a urine analysis system configured by a separate urine formed component measurement apparatus and biochemical measurement apparatus.

Although the above embodiment is described by way of example in which the measurement data obtained by the urine formed component measurement unit and the measurement data obtained by the biochemical measurement unit are analyzed by a single data analysis unit, the present invention is not limited to this example. As shown in the modification of FIG. 9, the measurement data obtained by the urine formed component measurement unit and the measurement data obtained by the biochemical measurement unit also may be respectively analyzed by separate computers (data analysis units). In this case, the diagnostic support information related to renal disease (urological disease) also may be obtained and output by the host computer (system controller) connected to the respective data analysis units. The diagnostic support apparatus for renal disease also may be applied to a first data analysis unit, so that the analysis results from the second data analysis unit can be obtained and the diagnostic support information can be output (displayed) by the first data analysis unit.

Although the above embodiment has been described by way of example in which the data analysis unit determines whether a measurement is required by the biochemical measurement unit based on the red blood cell concentration of the urine formed component analysis result when a user has not issued a measurement instruction to the biochemical measurement unit, the present invention is not limited to this example. As shown in the modification of FIG. 9, a system controller (host computer) rather than the data analysis unit also may determine whether a measurement is required by the biochemical measurement unit (biochemical measurement apparatus) when a user has not issued a measurement instruction to the biochemical measurement unit (biochemical measurement apparatus).

Although the above embodiment has been described by way of example in which the data analysis unit receives measurement instructions for the respective urine formed component measurement unit and biochemical measurement unit, the present invention is not limited to this example. the urine formed component measurement unit and the biochemical measurement unit may both normally perform measurements without receiving a measurement instruction. In this case, the diagnostic support information related to renal disease (urological disease) may be normally output.

Although the above embodiment has been described by way of example in which the CPU 411 of the data analysis unit 4 obtains diagnostic support information based on the magnitudes of the micro total protein estimated excretion value (m-TP), micro albumin estimated excretion value (m-ALB), and micro cholesterol estimated excretion value (m-CHO) as the quantitative value information of the present invention, the present invention is not limited to this example. The CPU 411 of the data analysis unit 4 also may obtain diagnostic support information based on the magnitude of the micro total protein concentration, micro albumin concentration, and micro cholesterol concentration as the quantitative value information of the present invention.

Although the above embodiment has been described by way of example in which the CPU 411 of the data analysis unit 4 determines the uniformity of red blood cells based on the red blood cell particle distribution information, the present invention is not limited to this example. The CPU 411 of the data analysis unit 4 also by determine the uniformity of red blood cells based on captured images of the red blood cells in the urine sample.

**Claims**

1. A diagnostic support apparatus for renal disease, comprising:

    a red blood cell information obtainer for obtaining at least one of red blood cell numerical information representing the number of red blood cells in a urine sample, and red blood cell morphological information representing morphology of the red blood cells in the urine sample; a quantitative value information obtainer for obtaining at least one of quantitative value information representing amount of protein component in the urine sample, and quantitative value information representing amount of lipid component in the urine sample; and

    a diagnostic support information output unit for outputting diagnostic support information related to renal disease based on information obtained by the red blood cell information obtainer and information obtained by the quantitative value information obtainer.

2. The diagnostic support apparatus of claim 1, wherein the protein component comprises at least one of total protein and albumin; and the lipid component comprises cholesterol.

3. The diagnostic support apparatus of claim 2, wherein the renal disease diagnostic support information comprises renal disease diagnostic support information related to suspected renal disease; and the diagnostic support information output unit outputs the renal disease diagnostic support information related to suspected renal disease, based on the information obtained by the red blood cell information obtainer and the information obtained by the quantitative value information obtainer.

4. The diagnostic support apparatus of claim 3, wherein the renal disease diagnostic support information related to suspected renal disease comprises renal disease diagnostic support information indicating that renal disease is suspected; the quantitative value information obtainer obtains albumin quantitative value information representing amount of albumin in the urine sample, and total protein quantitative value information representing amount of total protein in the urine sample; and the diagnostic support information output unit outputs the renal disease diagnostic support information indicating that renal disease is suspected, based on the albumin quantitative value information and the total protein quantitative value information.

5. The diagnostic support apparatus of claim 4, wherein the diagnostic support information output unit is configured to perform operations comprising:

    obtaining a first numerical value representing the amount of albumin in the urine sample based on the albumin quantitative value information; obtaining a second numerical value representing the amount of total protein in the urine sample based on the total protein quantitative value information; and outputting the renal disease diagnostic support information indicating that renal disease is suspected, when at least either the first numerical value exceeds a first threshold value or the second numerical value exceeds a second threshold value, or when the first numerical value is under the first threshold value, the second numerical value is under the second threshold value and the information obtained by the red blood cell information obtainer meets a predetermined condition.

6. The diagnostic support apparatus of claim 5, wherein the diagnostic support information output unit outputs the renal disease diagnostic support information indicating that renal disease is suspected, when the first numerical value is under the first threshold value, the second numerical value is under the second threshold value, the number of the red blood cells represented by the red blood cell numerical information exceeds a third threshold value and the morphology of the red blood cells represented by the red blood cell morphologic information does not indicate an approximately uniform morphology.

7. The diagnostic support apparatus of any one of claims 4 to 6, wherein the renal disease diagnostic support information indicating that renal disease is suspected comprises at least one of information indicating a degree of suspicion of renal disease and information related to a progression of renal disease; the red blood cell information obtainer obtains the red blood cell numerical information; the quantitative value information obtainer obtains cholesterol quantitative value information representing amount of cholesterol in the urine sample; and the diagnostic support information output unit outputs at least one of the information indicating the degree of suspicion of renal disease and information related to the progression of renal disease, based on at least one of the albumin quantitative value information and the total protein quantitative value information, the cholesterol quantitative value information and the red blood cell numerical information.

8. The diagnostic support apparatus of any one of claims 1 to 7, wherein the diagnostic support information output unit further outputs urologic disease diagnostic support information related to suspected urologic disease other than renal disease based on the information obtained by the red blood cell information obtainer and the information obtained by the quantitative value information obtainer.

9. The diagnostic support apparatus of claim 8, wherein the urologic disease diagnostic support information comprises urologic disease diagnostic support information indicating that urologic disease is suspected; the red blood cell information obtainer obtains the red blood cell morphological information; and the diagnostic support information output unit outputs the urologic disease diagnostic support information indicating that urologic disease is suspected, based on the red blood cell morphological information.

10. The diagnostic support apparatus of claim 9, wherein the diagnostic support information output unit outputs the urologic disease diagnostic support information indicating that urologic disease is suspected, when the morphology of the red blood cells represented by the red blood cell morphologic information indicates an approximately uniform morphology.

11. The diagnostic support apparatus of any one of claims 1 to 10, wherein the quantitative value information obtainer obtains creatinine quantitative value information representing amount of creatinine in the urine sample; and the diagnostic support information output unit outputs the renal disease diagnostic support information based on corrected result obtained by correcting the amount of at least one of the lipid component and the protein component in the urine sample by creatinine concentration in the urine sample.

12. The diagnostic support apparatus of any one of claims 1 to 11, wherein the diagnostic support information output unit comprises:

a display; and
a display controller for controlling the display so as to display a screen showing the renal disease diagnostic support information.

13. The diagnostic support apparatus of claim 12, wherein the display controller controls the display so as to display the screen comprising a first display region for displaying analysis result of the information obtained by the red blood cell information obtainer, a second display region for displaying analysis result of the information obtained by the quantitative value information obtainer, and a third display region for displaying the renal disease diagnostic support information.

14. A computer program product, comprising:

a computer readable medium, and
software instructions, on the computer readable medium, for enabling a computer to perform predetermined operations, comprising:

obtaining first data comprising at least one of the number of red blood cells in a urine sample, and morphology of the red blood cells in the urine sample;
obtaining second data comprising at least one of amount of protein component in the urine sample, and amount of lipid component in the urine sample; and
obtaining renal disease diagnostic support information related to renal disease based on the first data and the second data.

Fig. 1

# Fig. 2

# Fig. 3

# Fig. 4

Data analysis unit

# Fig. 5

# Fig. 6

Urine sample analysis process flow

```
                        ( START )
                            │
                    ┌───────────────────┐
                    │ Receive sample ID │──S1
                    └───────────────────┘
                            │
                        ╱ Urine formed ╲          No              ╱                    ╲   No
                    ╱ component measurement ╲──────────────▶╱ External data received? ╲────────┐
                        ╲     order?      ╱  S2              ╲                    ╱   S3        │
                            ╲   ╱                                    ╲   ╱                      │
                            │Yes                                     │Yes                       │
                            │                          ┌────────────────────────────┐          │
                            │                          │ Determine necessity of urine│──S4      │
                            │                          │ formed component            │          │
                            │                          │ measurement based on external│         │
                            │                          │ data                        │          │
                            │                          └────────────────────────────┘          │
                            │                                       │                           │
                            │                                   ╱ Urine formed ╲   S5           │
                            │◀──────────────────────────╱ component measurement ╲───No──────────┤
                    S6  ┌────────────────────────┐      ╲      required?      ╱                 │
                     ╲__│ Urine formed component │       ╲   ╱                                  │
                        │ measurement instruction│         │Yes                                 │
                        └────────────────────────┘         │                                    │
                            │                               │                                   │
                    S7  ┌────────────────────────┐          │                                   │
                     ╲__│ Urine formed component │◀─────────┘                                   │
                        │ measurement data       │                                              │
                        └────────────────────────┘                                             │
                            │                                                                   │
                    S8  ┌────────────────────────┐                                              │
                     ╲__│ Obtain urine formed    │                                              │
                        │ component analysis     │                                              │
                        │ results                │                                              │
                        └────────────────────────┘                                             │
                    S9      │                                                                   │
                     ╲  ╱ Biochemical ╲          No                                             │
                    ╱ measurement order? ╲──────────────────┐                                  │
                        ╲            ╱                       │                                  │
                            ╲   ╱                            ▼                                  │
                            │Yes                ┌──────────────────────────┐                   │
                            │                   │ Determine the necessity of│──S10             │
                            │                   │ biochemical measurement based│                │
                            │                   │ on urine formed component │                  │
                            │                   │ analysis results          │                  │
                            │                   └──────────────────────────┘                  │
                            │                              │                                    │
                            │                          ╱ Biochemical quantitative ╲   S11       │
                            │◀─────────────────╱ measurement required? ╲───No──────────────────┤
                   S12  ┌────────────────────────┐    ╲            ╱                            │
                    ╲__│ Biochemical quantitative│      ╲   ╱                                   │
                       │ measurement instruction │        │Yes                                  │
                       └────────────────────────┘         │                                     │
                            │                              │                                     │
                   S13  ┌────────────────────────┐         │                                     │
                    ╲__│ Receive biochemnical    │◀────────┘                                     │
                       │ quantitative            │                                              │
                       │ measurement data        │                                              │
                       └────────────────────────┘                                              │
                            │                                                                   │
                   S14  ┌────────────────────────┐                                              │
                    ╲__│ Obtain biochemical      │                                              │
                       │ quantitative analysis   │                                              │
                       │ results                 │◀─────────────────────────────────────────────┘
                       └────────────────────────┘
                   S15      │
                     ╱ Both urine formed ╲
                 ╱ component analysis results and ╲      No
                ╱ biochemical quantitative measurement ╲────────┐
                 ╲       results obtained?      ╱                │
                     ╲                    ╱                      │
                            │Yes                                 │
                    ┌────────────────────────────────┐  S16     │
                    │ Obtain diagnostic information   │──        │
                    └────────────────────────────────┘          │
                            │◀────────────────────────────────────┘
                    ┌────────────────────────┐  S17
                    │ Display analysis results│──
                    │ and diagnostic support  │
                    │ screen                  │
                    └────────────────────────┘
                            │
                        (  END  )
```

# Fig. 7

Diagnostic support information
obtaining process flow

START

S161 — m-TP<P ? — No / Yes

S162 — m-ALB<M1 ? — No / Yes

S163 — RBC concentration < L1 ? — No / Yes

S168 — m-CHO<N ? — No / Yes

S165 — Uniform RBC ? — No / Yes

S169 — RBC concentration < L2 ? — No / Yes

S174 — RBC concentration < L2 ? — No / Yes

S171 — Uniform RBC ? — No / Yes

S176 — Uniform RBC ? — No / Yes

S164 — Determination: no diagnostic support

S166 — Determination: urologic disease (suspected)

S167 — Determination: renal disease (suspected)

S170 — Determination: nonprogressive renal disease (suspected)

S172 — Determination: urologic disease (suspected); renal disease (suspected)

S173 — Determination: renal disease (suspected)

S175 — Determination: progressive renal disease (suspected)

S177 — Determination: urologic disease (suspected); renal disease (strongly suspected)

S178 — Determination: renal disease (strongly suspected)

RETURN

22

# Fig. 8

EP 2 239 568 A1

| File(F) | Edit(E) | View(V) | Data operations(R) | Action(A) | Output(P) | Settings(S) | Window(W) | Help(H) |

| F1 | F2 | F3 | F4 | F5 | F6 | F7 | F8 | F9 | F10 | F11 | F12 | Button 1 | Button 2 | Button 3 | Button 4 | Button 5 |

**REVIEW** (421a)

Not Validated (421)

| No. | 1 |
| | 263-03 |
| | 2005/04/22 09:54:17 |
| | ggggg |

| ID | 1 | ☺1974/02/05(M) |
| | Physician 1 | Ward 2 |
| | | Comment |

OO × × × (421g)

## Qualitative data (421b)
Current / Previous

| GLU | – | – |
| PRO | – | – |
| BLD | ± | + |
| DIL | – | – |
| URO | – | – |
| pH | 7 | 7 |
| KET | – | – |
| NIT | + | ++ |
| LEU | – | – |
| CRE | – | – |

## Formed component analysis (421c)
Current / Previous

RBC
7.5 /μL    9.5 /μL
WBC
15.0 /μL   30.0 /μL
EC
0.8 /μL    2.4 /μL
CAST
0.12 /μL   0.24 /μL
BACT
5.0 /μL    30.0 /μL

| RBC-Info. | RBC-Info. |
| [deformed?] | [deformed?] |

## Diagnostic support information (421f)

[Previous]
None

[Current]
renal disease (suspected)
<nonprogressive>

## Quantitative data (421e)
Current / Previous

| TP | 6.5 mg/dL | 6.0 mg/dL |
| ALB | 1.1 mg/dL | 1.0 mg/dL |
| CHO | 0.7 mg/dL | 0.6 mg/dL |
| CRE | 0.1 mg/dL | 0.1 mg/dL |

## Comment (421d)
Current / Previous

| Comment 1(z) | | re-exam required |
| Comment 2(x) | | |
| Comment 3(c) | | |

SED

HC

# Fig. 9

| | Europäisches Patentamt European Patent Office Office européen des brevets | **EUROPEAN SEARCH REPORT** | Application Number EP 10 15 8139 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2006/073606 A1 (FUKUDA MASAKAZU [JP]) 6 April 2006 (2006-04-06) * paragraph [0027] - paragraph [0033]; figures 1-4 * * paragraph [0043] - paragraph [0045]; figure 8 * ----- | 1-14 | INV. G01N33/493 G01N33/68 |
| Y | US 2006/084175 A1 (COMPER WAYNE [US]) 20 April 2006 (2006-04-20) * paragraphs [0001], [0021], [0032]; figures 1,2 * ----- | 1-14 | |
| A | US 2001/053551 A1 (JIANG MEIYI [JP] ET AL) 20 December 2001 (2001-12-20) * paragraph [0013] - paragraph [0029] * ----- | 1-14 | |
| A | EP 1 255 114 A1 (INT REAGENTS CORP [JP] SYSMEX CORP [JP]) 6 November 2002 (2002-11-06) * paragraph [0008] - paragraph [0021] * ----- | 1-14 | |
| A | EP 1 857 804 A2 (SYSMEX CORP [JP]) 21 November 2007 (2007-11-21) * paragraph [0007] - paragraph [0012] * ----- | 1-14 | TECHNICAL FIELDS SEARCHED (IPC) G01N |

The present search report has been drawn up for all claims

| Place of search | Date of completion of the search | Examiner |
|---|---|---|
| Munich | 10 June 2010 | Schindler, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 15 8139

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2010

| Patent document cited in search report | | Publication date | Patent family member(s) | | Publication date |
|---|---|---|---|---|---|
| US 2006073606 | A1 | 06-04-2006 | JP 4436742 B2<br>JP 2006098233 A | | 24-03-2010<br>13-04-2006 |
| US 2006084175 | A1 | 20-04-2006 | CA 2585816 A1<br>EP 1810036 A1<br>WO 2006044811 A1 | | 27-04-2006<br>25-07-2007<br>27-04-2006 |
| US 2001053551 | A1 | 20-12-2001 | JP 2002005925 A | | 09-01-2002 |
| EP 1255114 | A1 | 06-11-2002 | AU 3223401 A<br>CN 1398350 A<br>WO 0159462 A1<br>US 2003017523 A1 | | 20-08-2001<br>19-02-2003<br>16-08-2001<br>23-01-2003 |
| EP 1857804 | A2 | 21-11-2007 | CN 101173888 A<br>JP 2007309728 A<br>US 2009050821 A1 | | 07-05-2008<br>29-11-2007<br>26-02-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5325168 A
- JP 2001228158 A